# EUROPÄISCHE PATENTSCHRIFT

(11) **EP 0 974 585 B1**
(45) Veröffentlichungstag und Bekanntmachung des Hinweises auf die Patenterteilung: **01.10.2003**
(21) Anmeldenummer: 99113419.8
(22) Anmeldetag: 12.07.1999
(51) Int. Cl.: C07D 231/12

(54) **Verfahren zur Herstellung von Pyrazolen**
Method for the preparation of pyrazoles
Procédé de préparation de pyrazoles

(30) Priorität: 15.07.1998 DE 19831656
(43) Veröffentlichungstag der Anmeldung: 26.01.2000
(73) Patentinhaber: BASF AKTIENGESELLSCHAFT, 67056 Ludwigshafen (DE)
(72) Erfinder: Barth, Thomas, Dr., 67067 Ludwigshafen (DE); Rieber, Norbert, Dr., 68259 Mannheim (DE); Erhardt, Klaus, Dr., 69181 Leimen (DE)

(56) Entgegenhaltungen:
- EP-A- 0 020 964
- EP-A- 0 418 845
- D.S. NOYCE ET AL.: "THE ULTRAVIOLET ABSORPTION SPECTRA OF SUBSTITUTED PYRAZOLES" JOURNAL OF ORGANIC CHEMISTRY, Bd. 20, 1955, Seiten 1681-1686, XP002124311 EASTON US
- A.J. VAN DER ZEEUW ET AL.: "THE FORMULATION OF KETONES" RECUEIL DES TRAVAUX CHIMIQUES DES PAYS-BAS., Bd. 84, 1965, Seiten 1535-1554, XP002124312 AMSTERDAM NL

## Beschreibung

Die vorliegende Erfindung betrifft ein Verfahren zur Herstellung von Pyrazolen durch Umsetzung von Carbonylverbindungen mit Ameisensäureestern oder Kohlenmonoxid und anschließender Umsetzung des Zwischenproduktes mit einem Hydrazin (R₃-NH-NH₂), wobei man das Zwischenprodukt abfiltriert oder abzentrifugiert.

Aus J. Org. Chem. (1955), Vol. 20, Seite 1683 - 1686 und Recl. Trav. Chim. Pays-Bas (1965), Vol. 84, Seiten 1535 - 1554 ist jeweils ein Verfahren zur Herstellung von 3-Formylbutan-2-on aus Ameisensäureestern oder Kohlenmonoxid und anschließende Umsetzung mit Hydrazin zum 3,4-Dimethylpyrazol bekannt. Diese Verfahren haben den Nachteil, daß als Nebenprodukt signifikante Mengen an 3-Ethylpyrazol entstehen, welches umständlich durch Destillation entfernt werden muß.

Ferner ist aus der DE-A-29 22 591 und der EP-A-418 845 jeweils ein Verfahren zur Herstellung von Pyrazolen durch Umsetzung von 1,3-Dicarbonylverbindungen mit Hydrazin bekannt.

Der vorliegenden Erfindung lag daher die Aufgabe zugrunde, ein Verfahren zur Herstellung von Pyrazolen zu entwickeln, bei dem isomere Pyrazole (wie z.B. beim 3,4-Dimethylpyrazol das 3-Ethylpyrazol) nicht umständlich entfernt werden müssen.

Demgemäß wurde ein neues und verbessertes Verfahren zur Herstellung von Pyrazolen der allgemeinen Formel I 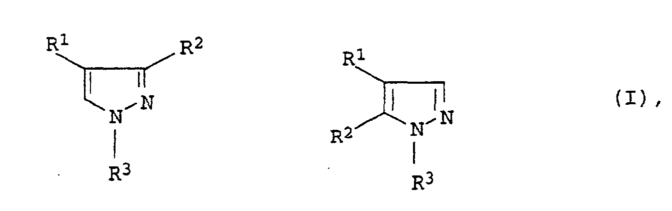 in der
- R¹,R²,R³: unabhängig voneinander C₁- bis C₂₀-Alkyl, C₃- bis C₈-Cycloalkyl, gegebenenfalls durch C₁- bis C₄-Alkyl, Halogen und/oder Nitro substituiertes Aryl oder C₇- bis C₂₀-Aralkyl,

- R¹,R³: zusätzlich unabhängig voneinander Wasserstoff
bedeuten, durch Umsetzung von einer Carbonylverbindung der allgemeinen Formel R¹-CH₂-CO-R², in der R¹ und R² die oben genannten Bedeutungen haben, in Gegenwart einer starken Base mit
a) Ameisensäureestern der allgemeinen Formel H-COOR⁴, in der R⁴ für C₁- bis C₈-Alkyl steht, bei Temperaturen von (-20) bis 70°C und einem Druck von 1 bis 50 bar oder
b) Kohlenmonoxid bei Temperaturen von 0 bis 100°C und einem Druck von 1,5 bis 150 bar
und Umsetzung des gebildeten Zwischenproduktes mit Hydrazinen der allgemeinen Formel R³-NH-NH₂, in der R³ die oben genannten Bedeutungen hat, in Gegenwart einer anorganischen oder organischen Säure bei Temperaturen von 0 bis 90°C und einem Druck von 1 bis 10 bar gefunden, welches dadurch gekennzeichnet ist, daß man das Zwischenprodukt abfiltriert oder abzentrifugiert.

Das erfindungsgemäße Verfahren läßt sich wie folgt durchführen:
a) Die Carbonylverbindungen der allgemeinen Formel R¹-CH₂-CO-R² können mit Ameisensäureestern der allgemeinen Formel H-COOR⁴ in Gegenwart starker Basen bei Temperaturen von (-20) bis 70°C und einem Druck von 1 (Atmosphärendruck) bis 50 bar, bevorzugt bei Temperaturen von 0 bis 60°C und einem Druck von 1 (Atmosphärendruck) bis 20 bar, besonders bevorzugt bei Temperaturen von 20 bis 50°C und Atmosphärendruck umgesetzt werden.
   Das Molverhältnis von Carbonylverbindung zum Ameisensäureester beträgt in der Regel von 0,1:1 bis 2:1, bevorzugt 0,2:1 bis 1,5:1, besonders bevorzugt 0,3:1 bis 1:1, insbesondere 0,6:1 bis 0,9:1. Das Molverhältnis von Carbonylverbindung zur starken Base beträgt in der Regel von 0,1:1 bis 2:1, bevorzugt 0,2:1 bis 1,5:1, besonders bevorzugt 0,3:1 bis 1:1, insbesondere 0,6:1 bis 0,9:1.
b) Die Carbonylverbindungen der allgemeinen Formel R¹-CH₂-CO-R² können mit Kohlenmonoxid durch Aufpressen im Überschuß in Gegenwart starker Basen bei Temperaturen von 0 bis 100°C und einem Druck von 1,5 bis 150 bar, bevorzugt bei Temperaturen von 20 bis 80°C und einem Druck von 5 bis 100 bar, besonders bevorzugt bei Temperaturen von 30 bis 70°C und einem Druck von 10 bis 40 bar umgesetzt werden.
   Das Molverhältnis von Carbonylverbindung zur starken Base beträgt in der Regel von 0,1:1 bis 2:1, bevorzugt 0,2:1 bis 1,5:1, besonders bevorzugt 0,3:1 bis 1:1, insbesondere 0,6:1 bis 0,9:1.
   In Ullmann's Enzyklopädie der technischen Chemie Vol. B 2 (Unit Operations I) finden sich in den Kapiteln 8 bis 11 Beschreibungen technischer Abläufe oder Apparaturen, mit denen Feststoffe abgetrennt werden können:
   - Seperation Processes: Centrifugal Sedimentation and Filtration (B 9-4 bis B 9-7)
   - Filter Selection (B 10-55 bis B 10-56)
   - Zentrifugen (B 11-5 bis B 11-19); Übersicht über Zentrifugen (B 11-6)

   Bei dem erfindungsgemäß beschriebenen Verfahren wird eine Abtrennung durch Filtration (mittels Druck oder Vakuum) oder Zentrifugieren besonders bevorzugt.
   Ganz besonders bevorzugt wird der kontinuierliche oder diskontinuierliche Einsatz von Rührdrucknutschen, Trommelfiltern (Druck oder Vakuum), leaf and plate filters, pressure plate filters oder Zentrifugen (wie in Schema B 11-6, Ullmann).
   Als starke Basen eignen sich Alkali- und Erdalkalimetallalkoholate oder Zinkalkoholate, bevorzugt Natriummethylat, Kaliummethylat, Natriumethylat, Kaliumethylat, Natriumisopropylat, Kaliumisopropoylat, Natriumbutylat, Kaliumbutylat, Natriumisobatylat, Kaliumisobutylat, Natriumtertbutylat oder Kaliumtertbutylat, besonders bevorzugt methanolisches Natriummethylat (NaOMe) oder Kaliummethylat (KOMe).

Als Lösungsmittel eignen sich Alkohole wie Methanol, Ethanol, Propanol, iso-Propanol, Butanol, iso-Butanol oder Ethylenglykol, Ether wie Diethylether, Tetrahydrofuran, Dioxan, bevorzugt Methanol, Ethanol oder Tetrahydrofuran, besonders bevorzugt Methanol oder Ethanol.

Das Zwischenprodukt, in der Regel ein Salz des entsprechenden Enolates, wird mit Hydrazinen der allgemeinen Formel R³-NH-NH₂, in der Regel als Hydrat, in Gegenwart einer anorganischen oder organischen Säure bei Temperaturen von 0 bis 90°C und einem Druck von 1 (Atmosphärendruck) bis 10 bar, bevorzugt bei Temperaturen von 10 bis 90°C und einem Druck von 1 (Atmosphärendruck) bis 5 bar, besonders bevorzugt bei Temperaturen von 30 bis 80°C und Atmosphärendruck in Wasser oder geeigneten organischen Lösungsmitteln oder deren Mischungen zu den Pyrazolen (I) umgesetzt.

Das Molverhältnis des Zwischenprodukts zum Hydrazin beträgt in der Regel von 0,25:1 bis 2:1, bevorzugt 0,5:1 bis 1,5:1, besonders bevorzugt 0,7:1 bis 1,25:1, insbesondere 0,9:1 bis 1,1:1. Das Molverhältnis des Zwischenprodukts zur anorganischen oder organischen Säure beträgt in der Regel von 0,1:1 bis 3,5:1, bevorzugt 0,3:1 bis 3:1, besonders bevorzugt 0,5:1 bis 2,5:1, insbesondere 0,7:1 bis 1,8:1.

Salze der Zwischenproduktenolate sind in der Regel Lithium-, Natrium-, Kalium-, Magnesium-, Calcium- oder Zinksalze, bevorzugt Lithium-, Natrium-, Kalium-, Magnesium- oder Calciumsalze, besonders bevorzugt Natrium- oder Kaliumsalze, insbesondere Natriumsalze.

Als anorganischen oder organischen Säuren eignen sich Schwefelsäure, Phosphorsäure, Alkyl- bzw. Arylsulfonsäuren, Ameisensäure, Essigsäure, Propionsäure, Buttersäure, Oxalsäure, Salzsäure, bevorzugt Salzsäure, Essigsäure, Ameisensäure, Schwefelsäure, besonders bevorzugt Schwefelsäure.

Als organische Lösungsmittel eignen sich Alkohole wie Methanol, Ethanol, Propanol, iso-Propanol, Butanol, iso-Butanol, Ethylenglykol oder Ether wie Diethylether, Tetrahydrofuran, Dioxan oder Dimethylethan, bevorzugt Methanol, Ethanol, Butanol, iso-Butanol, Ethylenglykol, Tetrahydrofuran oder Dimethoxyethan, besonders bevorzugt Methanol oder Ethanol. Bevorzugt sind Wasser und C₁- bis C₄-Alkohole wie Methanol, Ethanol, Propanol und Butanol, besonders bevorzugt in Wasser oder Methanol oder deren Mischungen.

Eine besondere Ausführungsform der Variante a) besteht darin, daß man die Gesamtmenge des Ameisensäureesters und der starken Base in zwei bis fünf Teile, bevorzugt zwei bis drei Teile aufteilt, besonders bevorzugt zweiteilt, wobei in der Regel der zweite Teil beider Edukte ca. 15 bis 50%, bevorzugt 20 bis 45%, besonders bevorzugt 25 bis 40%, insbesondere 25 bis 35% der Gesamtmenge beträgt. Beispielsweise werden hierbei zunächst je 1 bis 1.5 Äquivalente einer Carbonylverbindung, einer starken Base und 1.1 bis 1.7 Äquivalente eines Ameisensäureesters zusammengefügt und anschließend nochmals je 0.3 bis 1 Äquivalente desselben Ameisensäureesters und der starken Base nachdosiert.

Eine besondere Ausführungsform der Weiterverarbeitung besteht darin, daß das abfiltrierte und in Lösung gebrachte Zwischenprodukt in vorgelegtes Methanol, Wasser oder deren Mischungen gleichzeitig mit dem Hydrazin der allgemeinen Formel R³-NH-NH₂ und Schwefelsäure in einem Molverhältnis von 1 : (1 bis 1.5) : (1.5 bis 2), ganz besonders bevorzugt 1 : 1 : 1.25 zudosiert wird.

Die gleichzeitige Zudosierung des Zwischenproduktes, eines Hydrazins (R³-NH-NH₂) und der anorganischen oder organischen Säure kann so durchgeführt werden, daß die Komponenten in getrennten Vorlagebehältern vorliegen und im gewünschten Molverhältnis in den Reaktionsbehälter gegeben werden.

Das Zwischenprodukt kann nach Isolierung durch Filtration oder Zentrifugieren entweder in fester Form oder in Lösung weiterverarbeitet werden.

Vorteilhafterweise kann eine gewisse Menge eines Lösungsmittels oder Lösungsmittelgemisches im Reaktionsbehälter vorgelegt werden. Zusätzlich können die Reaktionskomponenten im gleichen Lösungsmittel gelöst werden.

In einer Variante kann auch zunächst die Mischung eines Hydrazins (R³-NH-NH₂) mit der anorganischen oder organischen Säure hergestellt werden. Diese Vormischung kann dann gleichzeitig mit dem Zwischenprodukt zudosiert werden.

Die Hydrazine (R³-NH-NH₂) und die anorganische oder organische Säure können auch in Form ihrer Additionssalze wie z.B. N₂H₄ HₙX (n von 1 bis 3) als Feststoff oder als Lösung zum Einsatz kommen.

Als symetrische oder unsymetrische Carbonylverbindungen der allgemeinen Formel R¹-CH₂-CO-R² eignen sich beispielsweise Aceton, Methylethylketon, Diethylketon, Methylpropylketon, Ethylpropylketon, Dipropylketon, 4-Methylpentan-2-on, Hexan-2-on oder 6-Methylheptan-2-on, bevorzugt unsymetrische Caronylverbindungen wie Methylethylketon, Methylpropylketon, Ethylpropylketon, 4-Methylpentan-2-on, Hexan-2-on oder 6-Methylheptan-2-on, besonders bevorzugt Methylethylketon, Methylpropylketon oder 6-Methylheptan-2-on, insbesondere Methylethylketon (Butan-2-on).

Als Ameisensäureester der allgemeinen Formel H-COOR⁴ eignen sich sich beispielsweise Methylformiat, Ethylformiat, Propylformiat, iso-Propylformiat, Butylformiat, iso-Butylformiat, Pentyl-, Hexyl-, Heptyl- oder Octylformiat, bevorzugt Methylformiat, Ethylformiat, Propylformiat, Propylformiat, iso-Propylformiat, Butylformiat oder iso-Butylformiat, besonders bevorzugt Methylformiat.

Als Hydrazine der allgemeinen Formel R³-NH-NH₂ eignen sich beispielsweise Hydrazin, Monoalkylhydrazine, Monoarylhydrazine, bevorzugt Hydrazin-Hydrat, Hydrazin, Methyl-, Phenyl- oder 2,4-Dinitrophenylhydrazin, besonders bevorzugt Hydrazin-Hydrat, Hydrazin, Methyl- oder Phenylhydrazin, insbesondere Hydrazin-Hydrat oder Hydrazin.

Durch das erfindungsgemäße Verfahren lassen sich Pyrazole (I) wie 3-Methylpyrazol, 3,4-Dimethylpyrazol, 3-Phenylpyrazol oder 3,4-Diphenylpyrazol, bevorzugt 3-Methylpyrazol oder 3,4-Dimethylpyrazol, besonders bevorzugt 3,4-Dimethyl-1H-pyrazol herstellen.

Die erfindungsgemäß beschriebenen Pyrazole (I) liegen für R³ = Wasserstoff in zwei tautomeren Formen vor:

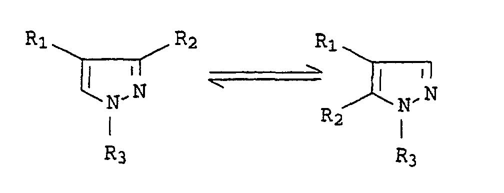

Die tautomere Form von 3,4-Dimethylpyrazol ist 4,5 Dimethylpyrazol.

Steht R³ nicht für Wasserstoff, so liegen keine tautomeren Pyrazole (I), sondern isomere Formen vor. Das Isomere zu 1,3,4-Trimethylpyrazol lautet 1,4,5-Trimethylpyrazol.

Die Substituenten R¹, R², R³ und R⁴ in den Pyrazolen (I), den Carbonylverbindungen, den Ameisensäureestern und den Hydrazinen haben folgende Bedeutungen:
- R¹,R²,R³: unabhängig voneinander
- C₁- bis C₂₀-Alkyl, bevorzugt C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, besonders bevorzugt C₁bis C₄-Alkyl wie Methyl, Ethyl, n-Propyl, iso-Propyl, n-Butyl, iso-Butyl, sec.-Butyl und tert.-Butyl, insbesondere Methyl,
- C₃- bis C₈-Cycloalkyl wie Cyclopropyl, Cyclobutyl, Cyclopentyl, Cyclohexyl, Cycloheptyl und Cyclooctyl, bevorzugt Cyclopentyl, Cyclohexyl und Cyclooctyl,
- Aryl wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl,
- C₇- bis C₂₀-Aralkyl, bevorzugt C₇- bis C₁₂-Phenylalkyl wie Benzyl, 1-Phenethyl, 2-Phenethyl, 1-Phenyl-propyl, 2-Phenyl-propyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl,
- durch C₁- bis C₄-Alkyl und/oder Halogen und/oder Nitro substituiertes Aryl wie ein- bis dreifach durch C₁- bis C₄-Alkyl und/oder Halogen und/oder Nitro substituiertes Phenyl (jedoch nicht mehr als zwei Nitrogruppen), bevorzugt 2-Methylphenyl, 3-Methylphenyl, 4-Methylphenyl, 2,4-Dimethylphenyl, 2,5-Dimethylphenyl, 2,6-Dimethylphenyl, 3,4-Dimethylphenyl, 3,5-Dimethylphenyl, 2,3,4-Trimethylphenyl, 2,3,5-Trimethylphenyl, 2,3,6-Trimethylphenyl, 2,4,6-Trimethylphenyl, 2-Ethylphenyl, 3-Ethylphenyl, 4-Ethylphenyl, 2-n-Propylphenyl, 3-n-Propylphenyl, 4-n-Propylphenyl, 2-Chlorphenyl, 3-Chlorphenyl, 4-Chlorphenyl, 2,4-Dichlorphenyl, 2,5-Dichlorphenyl, 2,6-Dichlorphenyl, 3,4-Dichlorphenyl, 3,5-Dichlorphenyl, 2,3,4-Trichlorphenyl, 2,3,5-Trichlorphenyl, 2,3,6-Trichlorphenyl, 2,4,6-Trichlorphenyl, 2-Bromphenyl, 3-Bromphenyl, 4-Bromphenyl, 2,4-Dibromphenyl, 2,5-Dibromphenyl, 2,6-Dibromphenyl, 3,4-Dibromphenyl, 3,5-Dinitrophenyl, 2,3,4-Tribromphenyl, 2,3,5-Tribromphenyl, 2,3,6-Tribromphenyl, 2,4,6-Tribromphenyl, 2-Nitrophenyl, 3-Nitrophenyl, 4-Nitrophenyl, 2,4-Dinitrophenyl, 2,5-Dinitrophenyl, 2,6-Dinitrophenyl, 3,4-Dinitrophenyl oder 3,5-Dinitrophenyl, bevorzugt 4-Methylphenyl, 2,4-Dimethylphenyl, 4-Chlorphenyl, 2,4-Dichlorphenyl, 4-Nitrophenyl oder 2,4-Dinitrophenyl, besonders bevorzugt 4-Methylphenyl, 4-Chlorphenyl oder 4-Nitrophenyl,
- durch C₁- bis C₄-Alkyl und/oder Halogen und/oder Nitro substituiertes C₇- bis C₂₀-Aralkyl wie ein- bis dreifach durch C₁- bis C₄-Alkyl und/oder Halogen und/oder Nitro substituiertes C₇- bis C₁₂-Phenalkyl (jedoch nicht mehr als zwei Nitrogruppen), bevorzugt 2-Methylphenyl-methyl, 3-Methylphenyl-methyl, 4-Methylphenyl-methyl, 2,4-Dimethylphenyl-methyl, 2,5-Dimethylphenyl-methyl, 2,6-Dimethylphenyl-methyl, 3,4-Dimethylphenyl-methyl, 3,5-Dimethylphenyl-methyl, 2,3,4-Trimethylphenyl-methyl, 2,3,5-Trimethylphenyl-methyl, 2,3,6-Trimethylphenyl-methyl, 2,4,6-Trimethylphenyl-methyl, 2-Ethylphenyl-methyl, 3-Ethylphenyl-methyl, 4-Ethylphenyl-methyl, 2-n-Propylphenyl-methyl, 3-n-Propylphenyl-methyl, 4-n-Propylphenyl-methyl, 2-Chlorphenyl-methyl, 3-Chlorphenyl-methyl, 4-Chlorphenyl-methyl, 2,4-Dichlorphenyl-methyl, 2,5-Dichlorphenyl-methyl, 2,6-Dichlorphenyl-methyl, 3,4-Dichlorphenyl-methyl, 3,5-Dichlorphenyl-methyl, 2,3,4-Trichlorphenyl-methyl, 2,3,5-Trichlorphenyl-methyl, 2,3,6-Trichlorphenyl-methyl, 2,4,6-Trichlorphenyl-methyl, 2-Bromphenyl-methyl, 3-Bromphenyl-methyl, 4-Bromphenyl-methyl, 2,4-Dibromphenyl-methyl, 2,5-Dibromphenyl-methyl, 2,6-Dibromphenyl-methyl, 3,4-Dibromphenyl-methyl, 3,5-Dinitrophenyl-methyl, 2,3,4-Tribromphenyl-methyl, 2,3,5-Tribromphenyl-methyl, 2,3,6-Tribromphenyl-methyl, 2,4,6-Tribromphenyl-methyl, 2-Nitrophenyl-methyl, 3-Nitrophenyl-methyl, 4-Nitrophenyl-methyl, 2,4-Dinitrophenyl-methyl, 2,5-Dinitrophenyl-methyl, 2,6-Dinitrophenyl-methyl, 3,4-Dinitrophenyl-methyl, 3,5-Dinitrophenyl-methyl oder 2,4,6-Trinitrophenylmethyl, bevorzugt 2,4,6-Trichlorphenyl, 2,4,6-Trimethylphenyl oder 2,4,6-Trinitrophenylmethyl, besonders bevorzugt 2,4,6-Trichlorphenylmethyl,
- R¹,R³: unabhängig voneinander
- Wasserstoff
- R⁴: - C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, bevorzugt C₁- bis C₄-Alkyl wie Methyl, Ethyl, n-Propyl, iso-Propyl, n-Butyl, iso-Butyl, sec.-Butyl und tert.-Butyl, besonders bevorzugt Methyl.

Die nach dem erfindungsgemäßen Verfahren herstellbaren Pyrazole I sind wertvolle Ausgangsstoffe bei der Herstellung von Farbstoffen, Pharmazeutika und insbesondere von Pflanzenschutzmitteln. 3,4-Dimethylpyrazol oder Säureadditionssalze hiervon sind wirksame Nitrifikationsinhibitoren.

### Beispiele

Die Gesamtausbeuten an 3,4-Dimethyl-1H-pyrazol wurden auf Butan-2-on bezogen.

### Beispiel 1

### Herstellung von 3,4-Dimethyl-1H-pyrazol mit Filtration des Zwischenproduktes

Zu 90 g (0,5 mol) einer 30%igen Natriummethylat-Lösung in Methanol wurde bei 30°C ein Gemisch aus 36 g (0,5 mol) Butan-2-on und 33 g (0,55 mol) Methylformiat zudosiert, 2 h bei 30°C nachgerührt, anschließend 27 g (0,15 mol) Natriummethylat-Lösung und 9 g (0,15 mol) Methylformiat nachdosiert und ca. 16 h bei Raumtemperatur nachgerührt. Die so erhaltene Suspension wurde abfiltriert und man erhielt 50 g (67 %) Natriumformylbutanon (Gehalt: 81,4 %).

50 g (0,33 mol) des festen Natriumformylbutanons wurden in 200 ml Wasser gelöst (Gehalt der Lösung: 16,3 %), bei Raumtemperatur vorgelegt, 21 g (0,21 mol) 96 %ige Schwefelsäure und anschließend 16,7 g (0,33 mol) Hydrazin-Hydrat (100 %) zugetropft und ca. 16 h bei Raumtemperatur nachgerührt. Der Reaktionsaustrag wurde mit 20 %iger Natronlauge auf pH 8,1 eingestellt. Nach Phasentrennung erhielt man 19,2 g (61 %) 3,4-Dimethyl-1H-pyrazol und 0,22 g (0,7 %) 3-Ethyl-1H-pyrazol. Die Gesamtausbeute an 3,4-Dimethyl-1H-pyrazol über beide Stufen betrug 40 %.

### Vergleichsbeispiel 1:

### Herstellung von 3,4-Dimethyl-1H-pyrazol unter Verwendung der gesamten Zwischenprodukt-Suspension ohne Filtration

Die Umsetzung wurde analog Beispiel 1 durchgeführt, jedoch wurde die erhaltene Suspension, die 0,41 mol Natriumformylbutanon enthielt (Ausbeute: 82 %), nicht filtriert, sondern direkt mit 50 %iger Schwefelsäure auf pH = 7 eingestellt und mit Hydrazinhydrat (100 %) analog zu Beispiel 1 umgesetzt. Der pH-Wert wurde während der Reaktion mit 50 %iger Schwefelsäure auf pH = 7 bis 8 korrigiert. Der Reaktionsaustrag wurde mit 20 %iger Natronlauge auf pH 8,1 eingestellt und abgedampft. Nach Aufnahme in 150 ml Wasser und Phasentrennung erhielt man 25,3 g (63 %) 3,4-Dimethyl-1H-pyrazol und 2,6 g (3 %) 3-Ethyl-1H-pyrazol. Die Gesamtausbeute an 3,4-Dimethyl-1H-pyrazol über beide Stufen betrug 52 %.

### Beispiel 2

### Herstellung von 3,4-Dimethyl-1H-pyrazol mit Filtration des Zwischenproduktes und gleichzeitiger Zudosierung bei der Umsetzung des Zwischenproduktes

Die Umsetzung wurde analog Beispiel 1 durchgeführt, jedoch wurde das in Wasser gelöste (Gehalt der Lösung: 15,4 %) Natriumformylbutanon (0,2 mol) bei Raumtemperatur mit 96 %iger Schwefelsäure (0,125 mol) und Hydrazin-Hydrat (80 %) (0,2 mol) gleichzeitig zu 50 ml Methanol zugetropft. Nach Einstellung des pH-Wertes auf 8,1 wurde das Methanol abgedampft. Nach Phasentrennung, zweimaliger Extraktion der wässrigen Phase mit je 50 ml tert. -Butylmethylether und Entfernen des Lösungsmittels erhielt man 17,2 g (90 %) 3,4-Dimethyl-1H-pyrazol. 3-Ethyl-1H-pyrazol war nicht nachweisbar. Die Gesamtausbeute an 3,4-dimethyl-1H-pyrazol über beide Stufen betrug 60 %.

### Vergleichsbeispiel 2

### Herstellung von 3,4-Dimethyl-1H-pyrazol ohne Filtration des Zwischenproduktes und mit gleichzeitiger Zudosierung bei der Umsetzung des Zwischenproduktes

Die Umsetzung wurde analog Beispiel 1 durchgeführt, jedoch wurde die erhaltene Suspension nicht filtriert, sondern in 60 ml Wasser gelöst. Man erhielt eine Lösung mit einem Natriumformylbutanon-Gehalt von 20,5 % (Ausbeute: 82 %). Ein 0,2 mol-Äquivaltent dieser Natriumformylbutanon-Lösung wurde gleichzeitig mit 96 %iger Schwefelsäure (0,125 mol) und Hydrazin-Hydrat (80 %) (0,2 mol) analog Beispiel 2 umgesetzt. Die Aufarbeitung erfolgte wie in Beispiel 2. Man erhielt 14,5 g (79 %) 3,4-Dimethyl-1H-pyrazol und 1,7 g (9,5 %) 3-Ethyl-1H-pyrazol. Die Gesamtausbeute an 3,4-Dimethyl-1H-pyrazol über beide Stufen betrug 65 %.

### Beispiel 3

### Carbonylierung von Butan-2-on

90 g (0.5 mol) einer 30 % igen Natriummethylat-Lösung in Methanol und 17 g (0.53 mol) Methanol wurden in einen Stahlautoklaven (300 ml) gefüllt, auf 70 °C erhitzt und 10 bar Kohlenmonoxid (CO) aufgepresst. Während der Autoklav unter Rühren auf Raumtemperatur abkühlte, wurde ein Kohlenmonoxid-Druck von 10 bar aufrecht erhalten. Nach beendeter Kohlenmonoxidaufnahme wurden bei Atmosphärendruck 36.1 g (0.5 mol) Butan-2-on zugegeben und 40 bar Kohlenmonoxid bis zum Erreichen der Druckkonstanz aufgepresst.

Nach beendeter Reaktion wurde der Reaktionsaustrag in Toluol aufgenommen und abfiltriert. Man erhielt 50 g (78%) eines farblosen Feststoffes, der nach Titration zu 94.7 % aus dem gewünschten Natrium-Enolat bestand.

### Vergleichsbeisspiel 3

### (analog Recl. Trav. Chim. Pays-Bas (1965), Vol. 84, Seite 1552)

3.3 g (64 mmol) Natriummethylat wurden in 50 ml Dimethoxyethan vorgelegt, 4.5 g (62 mmol) Butan-2-on und 3.7 g (62 mmol) Methylformiat zugegeben und über Nacht bei Raumtemperatur aufbewahrt. Nach Abfiltrieren und zweimaligem Waschen des Filterrückstand mit je 10 ml Diethylether erhielt man 7.3 g (63%, 39 mmol) Natriumenolat.

6.5 g (34,7 mmol) des Natriumenolats wurden in 40 ml Wasser gelöst, mit 3.7 g (62 mmol) Eisessig versetzt und 3.1 g (62 mmol) Hydrazin-Hydrat zugetropft. Nach 2 h Erhitzen unter Rückfluß wurde der zweiphasige Reaktionsaustrag mit Eisessig auf pH = 7 eingestellt, einmal mit 20 ml Methyl-tert.-butylether extrahiert, die organische Phase wird einmal mit 20 ml 10 %iger Natriumcarbonat-Lösung gewaschen und über Natriumsulfat getrocknet. Nach entfernen des Lösungsmittels erhielt man 2,1 g einer Mischung aus 95% 3,4-Dimethyl-1H-pyrazol (20,8 mmol, 60 %) und 4% 3-Ethyl-1H-pyrazol (bestimmt durch HPLC). Die Gesamtausbeute an 3,4-Dimethylpyrazol über beide Stufen betrug 38 %.

## Patentansprüche

1. Verfahren zur Herstellung von Pyrazolen der allgemeinen Formel I 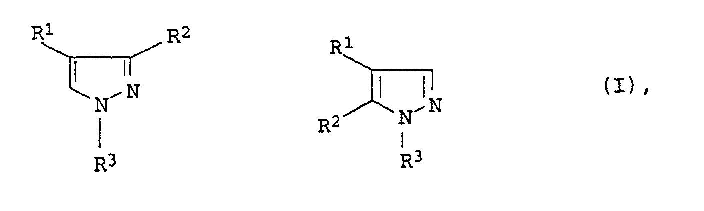 in der
R¹,R²,R³ unabhängig voneinander C₁- bis C₂₀-Alkyl, C₃- bis C₈-Cycloalkyl, gegebenenfalls durch C₁- bis C₄-Alkyl, Halogen und/oder Nitro substituiertes Aryl oder C₇- bis C₂₀-Aralkyl und
R¹,R³ zusätzlich unabhängig voneinander Wasserstoff
bedeuten, durch Umsetzung von einer Carbonylverbindung der allgemeinen Formel R¹-CH₂-CO-R², in der R¹ und R² die oben genannten Bedeutungen haben, in Gegenwart einer starken Base mit
a) Ameisensäureestern der allgemeinen Formel H-COOR⁴, in der R⁴ für C₁- bis C₈-Alkyl steht, bei Temperaturen von (-20) bis 70°C und einem Druck von 1 bis 50 bar oder
b) Kohlenmonoxid bei Temperaturen von 0 bis 100°C und einem Druck von 1,5 bis 150 bar
und Umsetzung des gebildeten Zwischenproduktes mit Hydrazinen der allgemeinen Formel R³-NH-NH₂, in der R³ die oben genannten Bedeutungen hat, in Gegenwart einer anorganischen oder organischen Säure bei Temperaturen von 0 bis 90°C und einem Druck von 1 bis 10 bar, **dadurch gekennzeichnet, daß** man das Zwischenprodukt abfiltriert oder abzentrifugiert.

2. Verfahren zur Herstellung von Pyrazolen I nach Anspruch 1, **dadurch gekennzeichnet, daß**
R¹,R²,R³ unabhängig voneinander C₁- bis C₈-Alkyl, Cyclopentyl, Cyclohexyl, gegebenenfalls ein- bis dreifach durch C₁- bis C₄-Alkyl, Halogen und/oder Nitro substituiertes Phenyl oder C₇- bis C₁₂-Aralkyl und
R¹,R³ zusätzlich unabhängig voneinander Wasserstoff
bedeuten.

3. Verfahren zur Herstellung von Pyrazolen I nach Anspruch 1, **dadurch gekennzeichnet, daß**
R¹,R² unabhängig voneinander C₁- bis C₄-Alkyl, Cyclopentyl, Cyclohexyl, Phenyl oder C₇- bis C₁₂-Aralkyl und
R¹,R³ zusätzlich unabhängig voneinander Wasserstoff
bedeuten.

4. Verfahren zur Herstellung von Pyrazolen I nach Anspruch 1, **dadurch gekennzeichnet, daß**
R¹,R² Methyl und
R³ Wasserstoff
bedeuten.

5. Verfahren zur Herstellung von Pyrazolen I nach den Ansprüchen 1 bis 4, **dadurch gekennzeichnet, daß** man die Umsetzung a) mit Ameisensäureestern bei Temperaturen von 0 bis 60°C und einem Druck von 1 bis 20 bar durchführt.

6. Verfahren zur Herstellung von Pyrazolen I nach den Ansprüchen 1 bis 5, **dadurch gekennzeichnet, daß** man bei der Umsetzung von Carbonylverbindungen mit Ameisensäureestern die Gesamtmenge an Base und Ameisensäureester in zwei bis fünf Teile aufteilt und nacheinander der Reaktionsmischung zugibt.

7. Verfahren zur Herstellung von Pyrazolen I nach den Ansprüchen 1 bis 5, **dadurch gekennzeichnet, daß** man bei der Umsetzung von Carbonylverbindungen mit Ameisensäureestern die Gesamtmenge an Base und Ameisensäureester in zwei bis drei Teile aufteilt und nacheinander der Reaktionsmischung zugibt.

8. Verfahren zur Herstellung von Pyrazolen I nach den Ansprüchen 1 bis 5, **dadurch gekennzeichnet, daß** man bei der Umsetzung von Carbonylverbindungen mit Ameisensäureester die Gesamtmenge an Base und Ameisensäureester in zwei Teile aufteilt und nacheinander der Reaktionsmischung zugibt.

9. Verfahren zur Herstellung von Pyrazolen I nach den Ansprüchen 1 bis 4, **dadurch gekennzeichnet, daß** man die Umsetzung b) mit Kohlenmonoxid bei Temperaturen von 20 bis 80°C und einem Druck von 5 bis 100 bar durchführt.

10. Verfahren zur Herstellung von Pyrazolen I nach den Ansprüchen 1 bis 8, **dadurch gekennzeichnet, daß** man die Umsetzung mit Hydrazinen bei Temperaturen von 10 bis 90°C und einem Druck von 1 bis 20 bar durchführt.

11. Verfahren zur Herstellung von Pyrazolen I nach den Ansprüchen 1 bis 10, **dadurch gekennzeichnet, daß** man das Zwischenprodukt, ein Hydrazin (R³-NH-NH₂) und die anorganische oder organische Säure gleichzeitig zudosiert.

## Claims

1. A process for preparing pyrazoles of the formula I 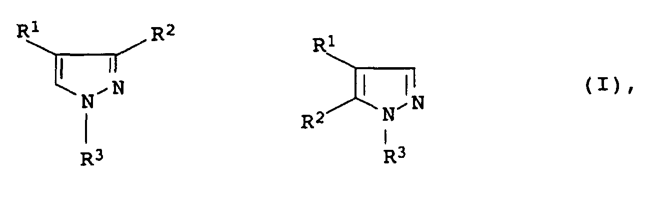 in which
R¹,R²,R³ independently of one another are C₁- to C₂₀-alkyl, C₃- to C₈-cycloalkyl, or C₇- to C₂₀-aralkyl or aryl, unsubstituted or substituted by C₁- to C₄-alkyl, halogen and/or nitro and
R¹,R³ are additionally independently of one another hydrogen,
by reacting a carbonyl compound of the formula R¹-CH₂-CO-R², in which R¹ and R² are as defined above, in the presence of a strong base with
a) formic esters of the formula H-COOR⁴, in which R⁴ is C₁-to C₈-alkyl at from (-20) to 70°C and a pressure of from 1 to 50 bar or
b) carbon monoxide at from 0 to 100°C and a pressure of from 1.5 to 150 bar
and reacting the resulting intermediate with hydrazines of the formula R³-NH-NH₂, in which R³ is as defined above, in the presence of an inorganic or organic acid at from 0 to 90°C and a pressure of from 1 to 10 bar, which comprises filtering off or centrifuging off the intermediate.

2. A process for preparing pyrazoles I as claimed in claim 1, wherein
R¹,R²,R³ independently of one another are C₁- to C₈-alkyl, cyclopentyl, cyclohexyl, or C₇- to C₁₂-aralkyl or phenyl, unsubstituted or mono- to trisubstituted by C₁- to C₄-alkyl, halogen and/or nitro and
R¹,R³ are additionally independently of one another hydrogen.

3. A process for preparing pyrazoles I as claimed in claim 1, wherein
R¹,R² independently of one another are C₁- to C₄-alkyl, cyclopentyl, cyclohexyl, phenyl or C₇- to C₁₂-aralkyl and
R¹,R³ are additionally independently of one another hydrogen.

4. A process for preparing pyrazoles I as claimed in claim 1, wherein
R¹,R² are methyl and
R³ is hydrogen.

5. A process for preparing pyrazoles I as claimed in any of claims 1 to 4, wherein the reaction a) with formic esters is carried out at from 0 to 60°C and a pressure of from 1 to 20 bar.

6. A process for preparing pyrazoles I as claimed in any of claims 1 to 5, wherein in the reaction of carbonyl compounds with formic esters the total amount of base and formic ester is divided into two to five parts and added successively to the reaction mixture.

7. A process for preparing pyrazoles I as claimed in any of claims 1 to 5, wherein in the reaction of carbonyl compounds with formic esters the total amount of base and formic ester is divided into two to three parts and added successively to the reaction mixture.

8. A process for preparing pyrazoles I as claimed in any of claims 1 to 5, wherein in the reaction of carbonyl compounds with formic esters the total amount of base and formic ester is divided into two and added successively to the reaction mixture.

9. A process for preparing pyrazoles I as claimed in any of claims 1 to 4, wherein the reaction b) with carbon monoxide is carried out at from 20 to 80°C and a pressure of from 5 to 100 bar.

10. A process for preparing pyrazoles I as claimed in any of claims 1 to 8, wherein the reaction with hydrazines is carried out at from 10 to 90°C and a pressure of from 1 to 20 bar.

11. A process for preparing pyrazoles I as claimed in any of claims 1 to 10, wherein the intermediate, a hydrazine (R³-NH-NH₂) and the inorganic or organic acid are metered in simultaneously.

## Revendications

1. Procédé de préparation de pyrazoles de formule générale I 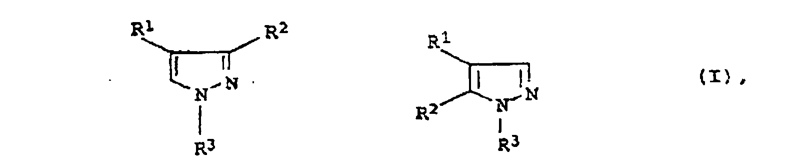 dans laquelle
R¹, R², R³ représentent indépendamment l'un de l'autre un alkyle en C₁ à C₂₀, un cycloalkyle en C₃ à C₈, le cas échéant par un aryle substitué par un alkyle en C₁ à C₄, un halogène et/ou un nitro, ou un aralkyle en C₇ à C₂₀ et
R¹, R³ représentent en outre indépendamment l'un de l'autre un hydrogène,
par réaction d'un composé carbonyle de formule générale R¹-CH₂-CO-R², dans laquelle R¹ et R² ont les significations données ci-dessus, en présence d'une base forte avec
a) des esters d'acide formique de formule générale H-COOR⁴, dans laquelle R⁴ représente un alkyle en C₁ à C₈, à des températures de -20 à 70°C et à une pression de 1 à 50 bars, ou
b) du monoxyde de carbone à des températures de 0 à 100°C et à une pression de 1,5 à 150 bars,
et par réaction du produit intermédiaire formé avec des hydrazines de formule générale R³-NH-NH₂, dans laquelle R³ possède les significations mentionnées, en présence d'un acide inorganique ou organique à des températures de 0 à 90°C et à une pression de 1 à 10 bars, **caractérisé en ce qu'**on filtre ou **en ce qu'**on centrifuge le produit intermédiaire

2. Procédé de préparation de pyrazoles I selon la revendication 1, **caractérisé en ce que**
R¹, R², R³ représentent indépendamment l'un de l'autre un alkyle en C₁ à C₈, un cyclopentyle, un cyclohexyle, un phényle éventuellement mono- à tri-substitué par un alkyle en C₁ à C₄, un halogène et/ou un nitro ou un aralkyle en C₇ à C₁₁ et
R¹, R³ représentent indépendamment l'un de l'autre un hydrogène

3. Procédé de préparation de pyrazoles I selon la revendication 1, **caractérisé en ce que**
R¹, R² représentent indépendamment l'un de l'autre un alkyle en C₁ à C₄, un cyclopentyle, un cyclohexyle, un phényle ou un aralkyle en C₇ à C₁₂ et
R¹, R³ représentent en outre indépendamment l'un de l'autre un hydrogène.

4. Procédé de préparation de pyrazoles I selon la revendication 1, **caractérisé en ce que** R¹, R² représentent un méthyle et R³ représentent un hydrogène.

5. Procédé de préparation de pyrazoles I selon les revendications 1 à 4, **caractérisé en ce qu'**on conduit la réaction a) avec des esters d'acide formique à des températures de 0 à 60°C et à une pression de 1 à 20 bars.

6. Procédé de préparation de pyrazoles I selon les revendications 1 à 5, **caractérisé en ce que** dans la réaction des composés carbonyles avec les esters d'acide formique on divise la quantité totale de base et d'ester d'acide formique en 2 à 5 parties et on les ajoute successivement au mélange réactionnel.

7. Procédé de préparation de pyrazoles I selon les revendications 1 à 5, **caractérisé en ce que** dans la réaction des composés carbonyles avec les esters d'acide formique, on divise la quantité totale de base et d'ester d'acide formique en deux à trois parties et on les ajoute successivement au mélange réactionnel.

8. Procédé de préparation de pyrazoles I selon les revendications 1 à 5, **caractérisé en ce que** dans la réaction de composés carbonyles avec les esters d'acide formique, on divise la quantité totale de base et d'ester d'acide formique en deux parties et **en ce qu'**on les ajoute successivement au mélange réactionnel.

9. Procédé de préparation de pyrazoles I selon les revendications 1 à 4, **caractérisé en ce qu'**on conduit la réaction b) avec le monoxyde de carbone à des températures de 20 à 80°C et à une pression de 5 à 100 bars.

10. Procédé de préparation de pyrazoles I selon les revendications 1 à 8, **caractérisé en ce qu'**on conduit la réaction avec les hydrazines à des températures de 10 à 90°C et à une pression de 1 à 20 bars.

11. Procédé de préparation de pyrazoles I selon les revendications 1 à 10, **caractérisé en ce qu'**on ajoute en quantités dosées le produit intermédiaire, une hydrazine (R³-NH NH₂) et l'acide inorganique ou organique de façon simultanée.
